# EUROPÄISCHE PATENTANMELDUNG

(11) **EP 4 047 397 A1**
(43) Veröffentlichungstag der Anmeldung: **24.08.2022**
(21) Anmeldenummer: 22155698.8
(22) Anmeldetag: 08.02.2022
(51) Int. Cl.: G01T 1/169

(54) **STRAHLUNGSMEDIZINISCHE DETEKTOREINRICHTUNG**

(30) Priorität: 18.02.2021 DE 102021103927
(71) Anmelder: PTW - Freiburg Physikalisch-Technische Werkstätten Dr. Pychlau GmbH, 79115 Freiburg (DE)
(72) Erfinder: Poppinga, Daniela, 13088 Berlin (DE); Lapp, Markus, 79211 Denzlingen (DE)
(74) Vertreter: Mertzlufft-Paufler, Cornelius

(57) **Zusammenfassung**

Strahlungsmedizinische Detektoreinrichtung (14), mit einer Detektoranordnung (1), die mehr als zwei Träger (2) aufweist, die miteinander gekreuzt angeordnet sind, und auf jedem Träger (2) ein Detektorfeld (7) angeordnet ist. (Fig. 1)

## Beschreibung

Die Erfindung betrifft eine strahlungsmedizinische Detektoreinrichtung mit einer Detektoranordnung.

Detektoreinrichtungen mit Detektoranordnung sind im Stand der Technik grundsätzlich bekannt. Eine solche Detektoreinrichtung kann beispielsweise in einem Phantom zur Bestimmung oder Überprüfung einer Strahlungsdosis oder eines Patientenplans verwendet werden. Die Detektoreinrichtung kann aber auch zur Ausmessung, insbesondere zur symmetrischen Charakterisierung und/oder Konstanzprüfung, eines strahlentherapeutischen Strahlungsgeräts verwendet werden.

Aufgabe der Erfindung ist es eine solche Detektoreinrichtung zu verbessern.

Diese Aufgabe wird durch eine Detektoreinrichtung mit den Merkmalen des Anspruch 1 gelöst.

Die erfindungsgemäße Detektoreinrichtung ist dadurch gekennzeichnet, dass die Detektoranordnung mehr als zwei Träger aufweist, die miteinander gekreuzt angeordnet sind, und dass auf jedem Träger ein Detektorfeld angeordnet ist. Auf diese Weise kann eine Detektoranordnung mit vielen Detektoren einfach hergestellt werden. Darüber hinaus kann die Detektoranordnung einfacher für verschiedene Auflösungen und Abmessungen angepasst werden, etwa in dem die Anzahl der gekreuzt angeordneten Träger verändert wird.

In einer Ausführung sind die Detektoren der Detektorfelder zur Bestimmung der Strahlungsdosis einer ionisierenden Strahlung, vorzugsweise Röntgenstrahlung, ausgebildet.

In einer Ausführung weisen die Träger jeweils Leiterplatten auf. Auf diese Weise ist eine elektrische Kontaktierung einfach durchführbar. Insbesondere kann der Träger durch die Leiterplatte gebildet sein.

In einer Ausführung durchdringen sich gekreuzte Träger gegenseitig und/oder definieren sich kreuzende Träger einen übereinstimmenden Detektionsbereich.

In einer Ausführung sind die gekreuzten Träger miteinander verklebt und/oder steckverbunden und/oder einstückig ausgebildet.

In einer Ausführung weisen die Träger einen vorzugsweise einseitig offenen Einschubschlitz auf, in den ein anderer Träger der mehr als zwei Träger eingesteckt ist, insbesondere mit einem oder seinem Einschubschlitz.

Insbesondere vorteilhaft kann es sein, wenn die Träger längs zum aufnehmenden Träger, also längs zur Achse, ineinandergesteckt sind. Auf diese Weise kann einfach erreicht werden, dass die Elektronik an einem gemeinsamen Ende der Detektoranordnung angeordnet ist.

In einer Ausführung ist auf einem Träger ein mechanischer und/oder elektrischer Steckplatz ausgebildet, in den ein anderer Träger, vorzugsweise quer zum aufnehmenden Träger, eingesteckt ist. Auf diese Weise ist die Detektoranordnung einfach zusammenbaubar.

In einer Ausführung bilden die mehr als zwei Träger ein zweidimensionales Gitter, insbesondere aus zwei gekreuzten Scharen, vorzugsweise parallel zueinander ausgerichteten Trägern.

In einer vorteilhaften Ausführung besitzen Träger einer Ausrichtung des Gitters lange Schlitze, die im Wesentlichen über die gesamte Länge des Trägers ausgedehnt sind, wobei die Träger jeweils nur einen schmalen, ungeschlitzten Bereich aufweisen. Die gekreuzt angeordneten Träger besitzen nur kurze Schlitze, insbesondere sind die Schlitze an den ungeschlitzten Bereich der anderen Träger angepasst, so dass diese ineinander gesteckt werden können. Dies hat den Vorteil, dass die Verteilung der Detektoren auf den Trägern einfach zu gestalten ist.

Der ungeschlitzte Bereich liegt zudem an einem Ende der Detektoranordnung, so dass Elektronik, die in diesem Bereich auf den Trägern angeordnet ist, einfach vor Strahlung geschützt werden kann, ohne dass ein Totraum entsteht, aus dessen Richtung keine Strahlung gemessen werden kann.

In einer Ausführung kreuzen sich die wenigstens zwei Träger in einer Schnittlinie, insbesondere wobei die Schnittlinie längs einer Einschubrichtung des Einschubs ausgerichtet ist.

In einer Ausführung sind die Träger an ihren zu der oder einer Einschubrichtung des Einschubs orientierten Stirnseite elektrisch kontaktiert.

In einer Ausführung sind die Träger jeweils planar ausgebildet.

In einer Ausführung sind die Zwischenräume zwischen den Trägern mit einem Material befüllt oder befüllbar, insbesondere wobei das Material zwischen den Trägern auswechselbar ist. Auf diese Weise kann eine bestimmte Absorptionsrate eingestellt werden, beispielsweise eine, die dem menschlichen Körper ähnlich ist.

In einer Ausführung ist die Detektoranordnung in einem Phantom angeordnet, vorzugsweise als, insbesondere gekapselter, Einschub.

In einer Ausführung ist das Phantom unregelmäßig geformt, insbesondere im Wesentlichen der Form eines menschlichen Kopfes nachgebildet oder angenähert ist.

In einer alternativen Ausführung ist das Phantom regelmäßig geformt, insbesondere wobei das Phantom als Rhombenkuboktaeder ausgebildet ist.

In einer Ausführung ist die Größe des Phantoms an die Größe eines menschlichen Kopfes angepasst.

In einer Ausführung ist das Phantom in der Detektoreinrichtung raumfest angeordnet.

In einer Ausführung ist das Phantom nicht deformierbar ausgebildet.

In einer Ausführung besitzt das Phantom eine wasserähnliche Dichte und/oder einen wasserähnlichen Schwächungskoeffizienten.

Die Erfindung umfasst auch eine Baureihe von strahlungsmedizinischen Detektoreinrichtungen, mit wenigstens zwei Varianten, die jeweils eine erfindungsgemäße strahlungsmedizinische Detektoreinrichtung bilden, wobei die wenigstens zwei Varianten unterschiedliche Außenkonturen aufweisen und die Detektoranordnungen als Einschub übereinstimmend ausgebildet sind. Auf diese Weise kann eine Detektoranordnung für verschiedene Einsatzzwecke einfach und kostengünstig konfiguriert werden.

Die Erfindung umfasst auch die Verwendung einer erfindungsgemäßen strahlungsmedizinischen Detektoreinrichtung zur Ausmessung eines strahlungsmedizinischen Strahlungsgeräts, insbesondere für eine dosimetrische Charakterisierung und/oder eine Konstanzprüfung, und/oder zur Überprüfung eines strahlungstherapeutischen Patientenplans.

Die Erfindung ist nachfolgend anhand bevorzugter Ausführungsbeispiele mit Bezug auf die beiliegenden Zeichnungen näher erläutert.

Es zeigt:
- Fig. 1:: eine Frontansicht einer Detektoranordnung einer erfindungsgemäßen Detektoreinrichtung mit gekreuzt angeordneten Trägern,
- Fig. 2:: einen Träger mit langen Schlitzen, wie er in der Fig. 1 horizontal angeordnet ist,
- Fig. 3:: einen Träger mit kurzen Schlitzen, wie er in der Fig. 1 vertikal angeordnet ist,
- Fig. 4:: einen Träger mit dargestellten Detektoren,
- Fig. 5:: einen Querschnitt durch einen Träger mit Detektoren,
- Fig. 6:: eine erfindungsgemäße Detektoreinrichtung mit einem regelmäßig geformten Phantom mit einem Einschub mit einer Detektoranordnung und
- Fig. 7:: eine erfindungsgemäße Detektoreinrichtung mit einem unregelmäßig geformten Phantom mit einem Einschub mit einer Detektoranordnung.

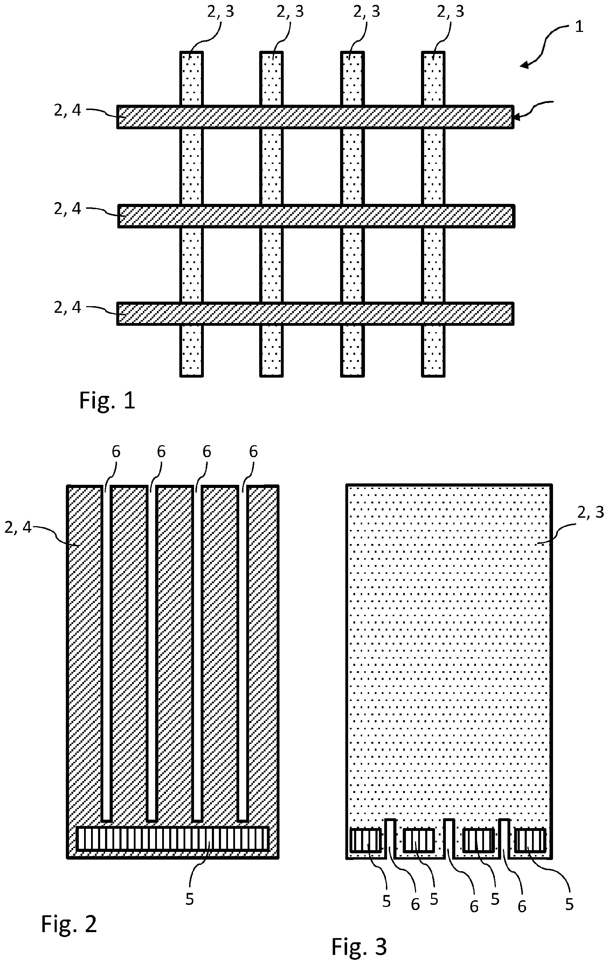

Die Fig. 1 zeigt schematisch eine Frontansicht einer Detektoranordnung 1 einer erfindungsgemäßen Detektoreinrichtung in Längsrichtung. Die Detektoranordnung 1 weist im Beispiel insgesamt sieben Träger 2 auf, wobei vier vertikale Träger 3 und drei horizontale Träger 4 abgebildet sind, die ein zweidimensionales Gitter bilden. Das abgebildete Beispiel dient hierbei nur zur übersichtlicheren Darstellung. Eine Detektoranordnung 1 kann in der Praxis in beiden Ausrichtungen auch mehr Träger aufweisen, beispielsweise sechs oder mehr horizontale und fünf oder mehr vertikale Träger. Die Anzahl der Träger kann dabei von den Abmessungen und der gewünschten Genauigkeit abhängen. Ebenfalls ist die Lage der Detektoreinrichtung nicht fest, so dass die Begriffe vertikal und horizontal nur zur Erläuterung des abgebildeten Beispiels dienen und nicht einschränkend auf die tatsächliche Lage der Träger verstanden werden soll. Demnach können die hier als vertikal gezeigten und bezeichneten Träger in einer Detektoreinrichtung tatsächlich auch horizontal oder diagonal ausgerichtet sein. Wichtig dabei ist jedoch die gekreuzte Anordnung der Träger.

Im Beispiel sind die vertikalen Träger 3 und die horizontalen Träger 4 in einem 90° Winkel zueinander gekreuzt. Es kann jedoch auch ein anderer Winkel gewählt werden.

In Fig. 2 ist einer der in Fig. 1 horizontalen Träger 4 in einer Draufsicht gezeigt.

Der Träger 4 weist an einem Ende einen Elektronikbereich 5 auf, in dem beispielsweise Ansteuer- und/oder Auswerteelektronik angeordnet sein kann.

Der Träger 4 weist im Beispiel vier Einschubschlitze 6 auf, die in Längsrichtung angeordnet sind und sich im Wesentlichen von dem Elektronikbereich 5 bis zum gegenüberliegenden Ende des Trägers 4 erstrecken. Die Einschubschlitze sind an diesem gegenüberliegenden Ende offen, so dass hier vertikale Träger 3 eingeschoben werden können. Dadurch wir auf einfache Weise eine gekreuzte Anordnung der Träger 2 erreicht.

Die Detektoren sind auf dem Träger außerhalb des Elektronikbereichs 5 angeordnet, in dieser Darstellung jedoch nicht gezeigt.

In Fig. 3 ist einer der in Fig. 1 vertikalen Träger 3 in einer Draufsicht gezeigt.

Der Träger 3 besitzt einen Elektronikbereich 5, dessen Ausdehnung in Längsrichtung im Wesentlichen dem horizontalen Träger 4 der Fig. 2 entspricht. Der vertikale Träger 4 weist ebenfalls Einschubschlitze 6 auf, die jedoch an dem Ende des Trägers 4 mit dem Elektronikbereich 5 offen sind und sich in Längsrichtung im Wesentlichen über den Elektronikbereich 5 erstrecken. Dadurch wird ein zu dem horizontalen Träger 4 komplementärer Einschubschlitz 6 gebildet. Beim Einschieben eines vertikalen Trägers 3 in einen horizontalen Träger 4 übergreift der Einschubschlitz 6 des vertikalen Trägers 3 den Elektronikbereich 5 des horizontalen Trägers 4. Die Elektronikbereiche 5 der Träger 3 und 4 liegen somit unmittelbar benachbart. Dadurch kann eine Kontaktierung der Elektronikbereiche 5 untereinander auf einfache Weise erfolgen. Hierzu können beispielsweise Steckverbinder verwendet werden.

Ein weiterer Vorteil dieser Anordnung ergibt sich daraus, dass sämtliche Elektronik in den Elektronikbereichen 5 an einem Ende der Detektoreinrichtung 1 angeordnet ist. Auf diese Weise ist es einfach möglich, die Elektronik vor der Strahlung zu schützen, beispielsweise in dem dieser Elektronikbereich 5 außerhalb eines Strahlungsbereichs angeordnet wird.

Abweichend von den hier gezeigten Ausführungen können die Einschubschlitze der Träger auch symmetrisch, also auf den horizontalen Träger 4 und den vertikalen Trägern 3 etwa gleich lang sein, oder andere abweichende Längenverhältnisse aufweisen.

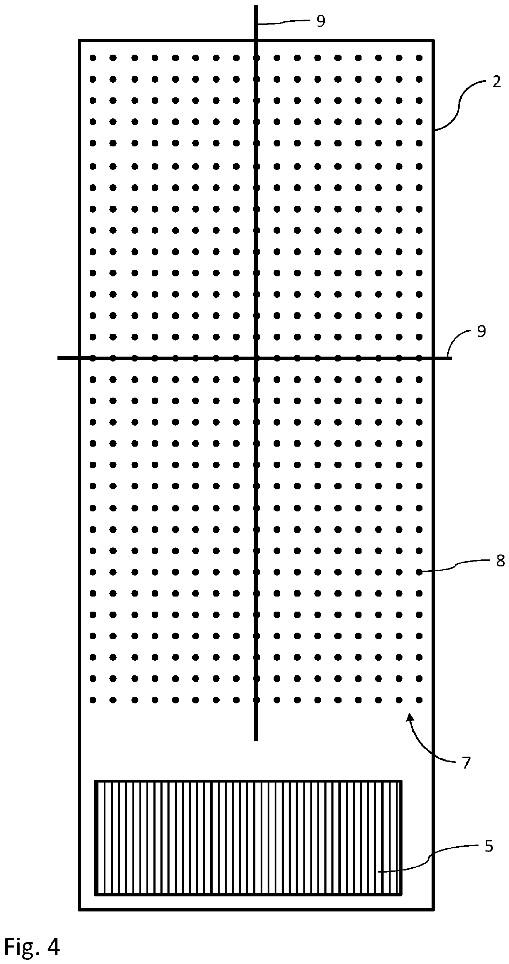

Die Fig. 4 zeigt schematisch einen Träger 2 mit einem Detektorfeld 7. In dieser Darstellung sind keine Einschubschlitze dargestellt. Der gezeigte Träger 2 kann ein vertikaler Träger 3 oder ein horizontaler Träger 4 sein, wobei jeweils den Fig. 2 oder 3 entsprechende oder andere Einschubschlitze 6 vorzusehen sind.

Das Detektorfeld 7 weist mehrere Detektoren 8 auf, die in einem regelmäßigen Raster angeordnet sind, das entlang der beiden Hauptachsen 9 des Trägers ausgerichtet ist. Im Beispiel weist das Detektorfeld 7 in Querrichtung jeweils 17 Detektoren 8 und in Längsrichtung jeweils 31 Detektoren 8 auf, insgesamt also 527. Das Detektorfeld 7 weist demnach eine sehr hohe Anzahl an Detektoren 8 auf. Dies ist insbesondere dadurch möglich, dass die dafür notwendige Ansteuerelektronik in dem Elektronikbereich direkt auf dem Träger 2 angeordnet ist. Insbesondere vorteilhaft ist es, wenn die Detektoren 8 mit einem Rasterabstand von 5 mm zueinander angeordnet sind. Dadurch kann eine hohe Auflösung der Messung erreicht werden, wodurch auch die Behandlung von Läsionen mit Abmessungen um 10 mm hinreichend genau überprüft werden können.

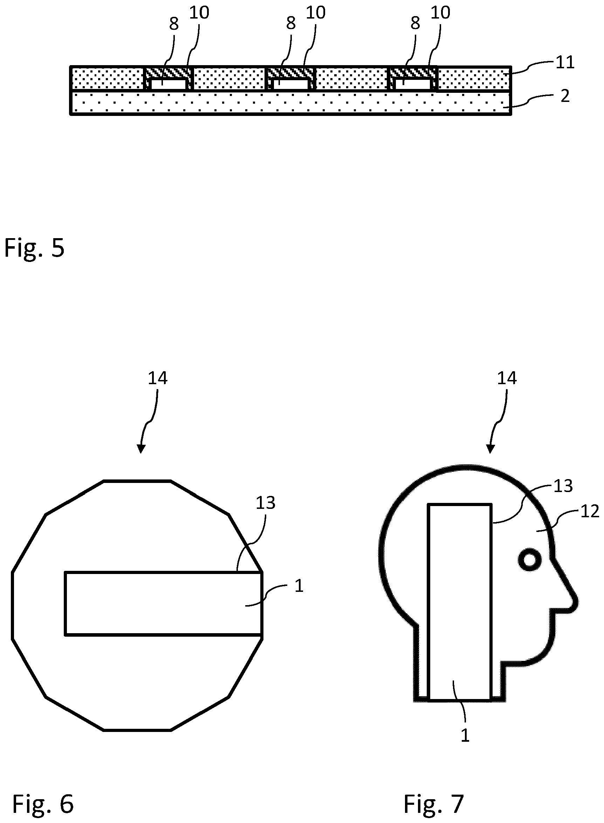

Die Fig. 5 zeigt einen Ausschnitt eines Querschnitts des Trägers 2 der Fig. 4. Der Träger 2 ist im Beispiel als mehrlagige Leiterplatte ausgebildet, so dass elektronische Bauteile, etwa im Elektronikbereich 5, und die Detektoren 8 direkt auf den Trägern 2 angeordnet sein können.

Die Detektoren 8 sind im Beispiel Dioden, die ohne eigenes Gehäuse direkt auf dem Träger 2 angeordnet sind. Die elektrische Kontaktierung der Dioden erfolgt dabei durch Drahtbonden und eventuell durch eine Leitpaste an der Unterseite der Dioden. Um die empfindlichen Bonddrähte zu schützen, sind die Detektoren 8 mit einer Vergussmasse 10 umgossen. Zwischen den Detektoren 8 ist ein Füllmaterial 11 angeordnet, so dass die Vergussmasse 10 nicht vollflächig auf dem Träger 2 angewandt werden muss. Die Vergussmasse 10 und das Füllmaterial 11 bilden eine ebene Oberfläche.

Die Breite der Einschubschlitze 6 entspricht daher im Wesentlichen der Gesamtdicke der Träger 2 inklusive dem Füllmaterial 11.

Die Fig. 6 zeigt eine erste Ausführung einer erfindungsgemäßen Detektoreinrichtung 14 mit einem regelmäßig geformten Phantom 12. Das Phantom 12 besitzt einen Einschub 13, in den beispielsweise eine Detektoranordnung 1 mit gekreuzten Trägern 2 wie oben beschrieben einsetzbar oder eingesetzt ist.

Die Fig. 7 zeigt eine zweite Ausführung einer erfindungsgemäßen Detektoreinrichtung 14 mit einem Phantom 12, das einem menschlichem Kopf nachempfunden ist. Dieses Phantom 12 besitzt ebenfalls einen Einschub 13 für eine Detektoranordnung.

Besonders vorteilhaft ist es, wenn die Phantome 12 der ersten und zweiten Ausführung übereinstimmende Einschübe 13 besitzen, so dass dadurch eine Baureihe von strahlungsmedizinischen Detektoreinrichtungen 14 mit wenigstens zwei Varianten gebildet ist, die jeweils eine erfindungsgemäße strahlungsmedizinische Detektoreinrichtung 14 bilden.

### Bezugszeichenliste

- 1: Detektoranordnung
- 2: Träger
- 3: vertikaler Träger
- 4: horizontaler Träger
- 5: Elektronikbereich
- 6: Einschubschlitz
- 7: Detektorfeld
- 8: Detektor
- 9: Hauptachse
- 10: Vergussmasse
- 11: Füllmaterial
- 12: Phantom
- 13: Einschub
- 14: Detektoreinrichtung

## Patentansprüche

1. Strahlungsmedizinische Detektoreinrichtung (14), mit einer Detektoranordnung (1), **dadurch gekennzeichnet, dass** die Detektoranordnung (1) mehr als zwei Träger (2) aufweist, die miteinander gekreuzt angeordnet sind, und dass auf jedem Träger (2) ein Detektorfeld (7) angeordnet ist.

2. Strahlungsmedizinische Detektoreinrichtung (14) nach Anspruch 1, **dadurch gekennzeichnet, dass** Detektoren (8) der Detektorfelder (7) zur Bestimmung der Strahlungsdosis einer ionisierenden Strahlung, vorzugsweise Röntgenstrahlung, ausgebildet sind.

3. Strahlungsmedizinische Detektoreinrichtung (14) nach einem der vorangehenden Ansprüche, **dadurch gekennzeichnet, dass** die Träger (2) jeweils Leiterplatten aufweisen.

4. Strahlungsmedizinische Detektoreinrichtung (14) nach einem der vorangehenden Ansprüche, **dadurch gekennzeichnet, dass** sich gekreuzte Träger (2) gegenseitig durchdringen und/oder dass sich kreuzende Träger (2) einen übereinstimmenden Detektionsbereich definieren.

5. Strahlungsmedizinische Detektoreinrichtung (14) nach einem der vorangehenden Ansprüche, **dadurch gekennzeichnet, dass** die gekreuzten Träger (2) miteinander verklebt und/oder steckverbunden und/oder einstückig ausgebildet sind.

6. Strahlungsmedizinische Detektoreinrichtung (14) nach einem der vorangehenden Ansprüche, **dadurch gekennzeichnet, dass** die Träger (2) einen vorzugsweise einseitig offenen Einschubschlitz (6) aufweisen, in den ein anderer Träger (2) der mehr als zwei Träger (2), vorzugsweise längs zum aufnehmenden Träger (2), eingesteckt ist, insbesondere mit einem oder seinem Einschubschlitz (6), und/oder dass auf einem Träger (2) ein mechanischer und/oder elektrischer Steckplatz ausgebildet ist, in den ein anderer Träger (2), vorzugsweise quer zum aufnehmenden Träger (2), eingesteckt ist.

7. Strahlungsmedizinische Detektoreinrichtung (14) nach einem der vorangehenden Ansprüche, **dadurch gekennzeichnet, dass** die mehr als zwei Träger (2) ein zweidimensionales Gitter, insbesondere aus zwei gekreuzten Scharen, vorzugsweise parallel zueinander ausgerichteten Trägern (2), bilden.

8. Strahlungsmedizinische Detektoreinrichtung (14) nach einem der vorangehenden Ansprüche, **dadurch gekennzeichnet, dass** sich die wenigstens zwei Träger (2) in einer Schnittlinie kreuzen, insbesondere wobei die Berührlinie längs einer Einschubrichtung des Einschubs ausgerichtet ist.

9. Strahlungsmedizinische Detektoreinrichtung (14) nach einem der vorangehenden Ansprüche, **dadurch gekennzeichnet, dass** die Träger (2) an ihren zu der oder einer Einschubrichtung des Einschubs orientierten Stirnseite elektrisch kontaktiert sind.

10. Strahlungsmedizinische Detektoreinrichtung (14) nach einem der vorangehenden Ansprüche, **dadurch gekennzeichnet, dass** die Träger (2) jeweils planar ausgebildet sind.

11. Strahlungsmedizinische Detektoreinrichtung (14) nach einem der vorangehenden Ansprüche, **dadurch gekennzeichnet, dass** die Zwischenräume zwischen den Trägern (2) mit einem Material befüllt oder befüllbar sind, insbesondere wobei das Material zwischen den Trägern (2) auswechselbar ist.

12. Strahlungsmedizinische Detektoreinrichtung (14) nach einem der vorangehenden Ansprüche, **dadurch gekennzeichnet, dass** die Detektoranordnung (1) in einem Phantom (12) angeordnet ist, vorzugsweise als insbesondere gekapselter Einschub (13) .

13. Strahlungsmedizinische Detektoreinrichtung (14) nach Anspruch 12, **dadurch gekennzeichnet, dass** das Phantom (12) unregelmäßig geformt ist, insbesondere im Wesentlichen der Form eines menschlichen Kopfes nachgebildet oder angenähert ist.

14. Strahlungsmedizinische Detektoreinrichtung (14) nach Anspruch 12, **dadurch gekennzeichnet, dass** das Phantom (12) regelmäßig geformt ist, insbesondere wobei das Phantom (12) als Rhombenkuboktaeder ausgebildet ist.

15. Strahlungsmedizinische Detektoreinrichtung (14) nach einem der vorangehenden Ansprüche 12 bis 14, **dadurch gekennzeichnet, dass** die Größe des Phantoms (12) an die Größe eines menschlichen Kopfes angepasst ist.

16. Strahlungsmedizinische Detektoreinrichtung (14) nach einem der vorangehenden Ansprüche 12 bis 15, **dadurch gekennzeichnet, dass** das Phantom (12) in der Detektoreinrichtung raumfest angeordnet ist.

17. Strahlungsmedizinische Detektoreinrichtung (14) nach einem der vorangehenden Ansprüche 12 bis 16, **dadurch gekennzeichnet, dass** das Phantom (12) nicht deformierbar ausgebildet ist.

18. Strahlungsmedizinische Detektoreinrichtung (14) nach einem der vorangehenden Ansprüche 12 bis 17, **dadurch gekennzeichnet, dass** das Phantom (12) eine wasserähnliche Dichte und/oder einen wasserähnlichen Schwächungskoeffizienten besitzt.

19. Baureihe von strahlungsmedizinischen Detektoreinrichtungen (14), mit wenigstens zwei Varianten, die jeweils eine strahlungsmedizinische Detektoreinrichtung (14) nach einem der vorangehenden Ansprüche bilden, **dadurch gekennzeichnet, dass** die wenigstens zwei Varianten unterschiedliche Außenkonturen aufweisen und dass die Detektoranordnungen (1) als Einschub (13) übereinstimmend ausgebildet sind.

20. Verwendung einer strahlungsmedizinischen Detektoreinrichtung (14) nach einem der Ansprüche 1 bis 18 zur Ausmessung eines strahlungsmedizinischen Strahlungsgeräts, insbesondere für eine dosimetrische Charakterisierung und/oder eine Konstanzprüfung, und/oder zur Überprüfung eines strahlungstherapeutischen Patientenplans.
